# EUROPÄISCHE PATENTSCHRIFT

(11) **EP 1 698 371 B1**
(45) Veröffentlichungstag und Bekanntmachung des Hinweises auf die Patenterteilung: **16.07.2008**
(21) Anmeldenummer: 05004835.4
(22) Anmeldetag: 04.03.2005
(51) Int. Cl.: A61M 39/28

(54) **Schnellschlussklemme zum Abklemmen von Schläuchen**
Quick acting tube shut-off clamp
Pince à action rapide pour obturer un tube souple

(43) Veröffentlichungstag der Anmeldung: 06.09.2006
(73) Patentinhaber: LIFEBRIDGE Medizintechnik AG, 84539 Ampfing (DE)
(72) Erfinder: Brieske, Gerhard, 84539 Ampfing (DE)
(74) Vertreter: Manitz, Finsterwald & Partner GbR

(56) Entgegenhaltungen:
- EP-A- 0 241 123
- EP-A- 0 718 001
- US-A- 4 105 028
- US-A- 4 596 374

## Beschreibung

Die Erfindung betrifft eine Schnellschlussklemme zum Abklemmen von Schläuchen.

In medizinischen Geräten, zum Beispiel Herz-Lungen-Maschinen werden Flüssigkeiten, insbesondere Blut, transportiert. Eine Herz-Lungen-Maschine dient u.a. dazu, die Funktion des Herzens und der Lunge eines Patienten während einer Herzoperation zu übernehmen und den Blutkreislauf aufrechtzuerhalten. Dabei ist es besonders wichtig, dass keine Luftblasen transportiert werden, um den Patienten nicht zu gefährden. Dazu ist in derartigen medizinischen Vorrichtungen an geeigneter Stelle ein Blasendetektor im Fluidkreislauf vorgesehen. Wird von diesem Detektor eine Luftblase im extrakorporalen Blutkreislauf festgestellt, so muss der Kreislauf sofort unterbrochen werden, damit eine Gefährdung des Patienten ausgeschlossen ist. Dazu dienen Klemmvorrichtungen, die an einem Schlauch des Fluidsystems angreifen.

Das Abdrücken des Schlauches durch die Klemmvorrichtung muss dabei äußerst schnell vonstatten gehen und sollte nur einige Millisekunden bis 100 Millisekunden Verzögerung aufweisen.

US 5,445,613 beschreibt eine Klemmvorrichtung, die entweder elektromagnetisch oder pneumatisch gegen die Federkraft einer Feder offen gehalten wird. Ein Ausschalten des Elektromagneten bzw. Abschalten des Luftdruckes ermöglichen ein Entspannen der Feder, das zu einem Schlie-βen der Klemmvorrichtung führt. Um die Klemmvorrichtung wieder zu öffnen, muss wiederum Strom durch den Elektromagneten geleitet werden oder die Pneumatik wieder mit Luftdruck beaufschlagt werden. Um die Klemmvorrichtung offen zu halten, muss hier also permanent Strom durch den Elektromagneten fließen oder der Luftdruck in der pneumatischen Vorrichtung aufrechterhalten werden, was insbesondere bei mobilen Einheiten aufgrund des hohen Energiebedarfs nachteilig ist.

DE 199 00 320 C1 beschreibt eine Vorrichtung, bei der eine Klemmbacke durch ein Halteelement gegen die Kraft einer Feder gehalten wird. Das Halteelement greift in eine Ausnehmung der Klemmbacke ein, wobei es in dieser Stellung durch eine elektromagnetische Vorrichtung gehalten wird. Solange die elektromagnetische Vorrichtung bestromt wird, hält das Halteelement die Klemmbacke. Ausschalten des Stromes löst das Halteelement von der Klemmbacke, die daraufhin von der Feder getrieben in ihre Verschlussstellung geht. Um die Klemmvorrichtung wieder zu öffnen wird die Klemmbacke von der gegenüberliegenden Seite mit Hilfe einer zweiten Vorrichtung zurückgeschoben.

EP 1132108 A1 beschreibt eine Vorrichtung, die mit Hilfe von Permanentmagneten zwei Gleichgewichtsstellungen für eine Klemmvorrichtung erzeugt. Eine dieser Gleichgewichtslagen entspricht der Verschlussstellung der Klemmvorrichtung, während die andere Gleichgewichtslage der Offenstellung entspricht. Mit Hilfe eines Elektromagneten wird die Klemmvorrichtung zwischen diesen zwei Gleichgewichtslagen hin- und herbewegt.

US-A-4,596,374 beschreibt eine Schnellschlussklemme, bei der ein Schlauch mit Hilfe einer Klemmbacke abgeklemmt wird. Mit Hilfe einer motorbetriebenen Spindel kann eine Klemmbackenanordnung derart nach oben bewegt werden, dass der Schlauch freigegeben wird. Die Rotationsbewegung der Spindel wird von einem Motor zur Verfügung gestellt, der je nach Bedarf mit Hilfe einer Kupplung an die Klemmbackenanordnung angekoppelt werden kann. Eine Auslösung zum Verschließen des Schlauches wird durch Entkupplung der Kupplung erreicht. Eine Torsionsfeder treibt dann die Spindel in entgegengesetzter Richtung, so dass die Klemmbacke gegen den Schlauch nach unten gezogen wird.

Aufgabe der vorliegenden Erfindung ist es, eine Schnellschlussklemme für transportable Einheiten anzugeben, deren Aufbau kompakt und sicher ist und die wenig Energie benötigt.

Diese Aufgabe wird mit einer Schnellschlussklemme mit den Merkmalen des Anspruchs 1 gelöst. Bevorzugte Ausführungsformen sind Gegenstand der Unteransprüche.

Eine erfindungsgemäße Schnellschlussklemme zum Abklemmen von Schläuchen weist eine Klemmbacke mit einer Klemmstellung auf, in der sie einen Schlauch abklemmen kann. Weiterhin weist die Klemmbacke eine Offenstellung auf. Es ist eine Haltevorrichtung vorgesehen, gegen die die Klemmbacke mit einer Federeinrichtung vorgespannt ist, so dass die Klemmbacke bei Entspannung der Feder in die Klemmstellung gebracht wird. Eine Verriegelungseinrichtung dient dazu, die Klemmbacke und die Haltevorrichtung gegen die Federkraft der Federeinrichtung in einer Verriegelungsstellung miteinander zu verriegeln. Schließlich weist die erfindungsgemäße Schnellschlussklemme einen Rückholmechanismus auf, mit dessen Hilfe die Klemmbacke mit der Haltevorrichtung aus der Klemmstellung in ihre Offenstellung zurückbewegt werden kann. Der Rückholmechanismus dient dabei zur Bewegung der Haltevorrichtung relativ zu der Klemmbacke bis die Haltevorrichtung und die Klemmbacke in ihrer Verriegelungsstellung zueinander sind. Diese Bewegung geschieht während die Klemmbacke in der Klemmstellung ist. Weiterhin dient der Rückholmechanismus dazu, die Haltevorrichtung im Anschluss daran zusammen mit der Klemmbacke in die Offenstellung der Klemmbacke zu überführen, nachdem die Klemmbacke und die Haltevorrichtung mit der Verriegelungseinrichtung miteinander verriegelt worden sind.

Mit der erfindungsgemäßen Schnellschlussklemme kann das schnelle Abklemmen des Schlauches sehr präzise und sicher ausgeführt werden. In der Offenstellung der Klemmbacke ist die Klemmbacke in der Haltevorrichtung verriegelt. Dabei ist sie gegen die Haltevorrichtung über die Federkraft der Feder vorgespannt. Detektiert zum Beispiel ein Blasendetektor in einer Herz-Lungen-Maschine eine Luftblase im Fluidsystem, so wird die Verriegelungseinrichtung gelöst. Sehr schnell, in der Regel innerhalb einiger Millisekunden bis 100 Millisekunden wird die Klemmbacke durch die Feder aus der Haltevorrichtung in die Klemmstellung gebracht. Die Klemmbacke presst in der Klemmstellung zum Beispiel einen Schlauch der Herz-Lungen-Maschine gegen eine Wand, z.B. die Rückwand eines Kanals, in dem der Schlauch geführt ist. Die Wand bildet dadurch ein passives Gegenlager für die Klemmbacke. Eine sehr schnelle Reaktion zum Verschluss des Schlauches ist also gewährleistet.

Mit der erfindungsgemäßen Vorrichtung kann die Klemmvorrichtung auf sehr einfache und im Wesentlichen mechanische Weise wieder zurück in die Offenstellung geführt werden. Dazu dient ein Rückholmechanismus, der die Haltevorrichtung relativ zur Klemmbacke so weit bewegt, bis die Klemmbacke und die Haltevorrichtung in ihrer Verriegelungsstellung zueinander sind. Während die Haltevorrichtung durch den Rückholmechanismus bewegt wird, verbleibt die Klemmbacke in ihrer Klemmstellung und stützt sich weiterhin gegen das passive Gegenlager ab.

Nachdem die Haltevorrichtung so weit bewegt worden ist, dass die Haltevorrichtung und die Klemmbacke in ihrer Verriegelungsstellung zueinander sind, wird die Verriegelungseinrichtung aktiviert, so dass die Klemmbacke und die Haltevorrichtung miteinander verriegelt sind. Mit Hilfe des Rückholmechanismus wird dann die Haltevorrichtung zusammen mit der Klemmbacke bewegt, so dass sich die Klemmbacke in ihre Offenstellung bewegt. Die erfindungsgemäße Schnellschlussklemme kann also auf sehr einfache und mechanische Weise in die Offenstellung zurückbewegt werden. Das System ist robust und wenig störanfällig.

Bereits während der Rückholmechanismus die Haltevorrichtung relativ zur Klemmbacke bewegt, wird die Federeinrichtung wieder gespannt. Während des Zurückbewegens der Haltevorrichtung zusammen mit der Klemmbacke steht also bereits die Auslöseenergie wieder voll zur Verfügung, so dass ggf. bereits während des Zurückbewegens die Klemmbacke wieder ausgelöst und der Schlauch erneut abgeklemmt werden kann. Es existiert somit kein Zeitraum, während dessen ein Verschluss des Schlauches nicht möglich ist.

Die Klemmbacke und die Haltevorrichtung können auf unterschiedliche Weise verschieblich zueinander gelagert sein. Eine sichere und einfache Ausgestaltung weist als Haltevorrichtung ein Hohlteil auf, in dem die Klemmbacke geführt wird.

Die Federeinrichtung ist derart mit der Haltevorrichtung und der Klemmbacke verbunden, dass sich bei Entspannung der Feder die Klemmbacke und die Haltevorrichtung auseinanderbewegen. Bei einer Ausführungsform mit einem Hohlteil als Haltevorrichtung kann dies einfach und auf leicht herzustellende Weise mit Hilfe von Ansätzen geschehen. Ein erster Ansatz ist dabei innerhalb des Hohlteils vorgesehen und ein zweiter Ansatz an der Außenseite der Klemmbacke. Zwischen diesen Ansätzen ist die Federeinrichtung verspannt.

Die Verriegelung von Haltevorrichtung und Klemmbacke zueinander erfolgt vorteilhafterweise mechanisch, um möglichst wenig energieverbrauchende Elemente betreiben zu müssen.

Eine einfache Ausgestaltung sieht dazu wenigstens ein Element vor, das im Verriegelungszustand seitlich aus der Klemmbacke in eine entsprechende Ausnehmung der Haltevorrichtung eingreift und in einem Entriegelungszustand die Ausnehmung freigibt. Es kann sich dabei zum Beispiel um Stäbe oder Kugeln handeln, die mechanisch aus der Klemmbacke herausgedrückt werden, um in die Ausnehmung der Haltevorrichtung einzugreifen. Eine vorteilhafte Ausführungsform umfasst eine oder mehrere Kugeln, die im verriegelten Zustand seitlich aus der Klemmbacke durch Öffnungen herausragen, die die Kugeln teilweise umfassen. Im entriegelten Zustand ragen die Kugeln nicht über die seitliche Ausdehnung der Klemmbacke hinaus und verriegeln die Klemmbacke somit nicht in der Haltevorrichtung.

Speziell bei einer mechanischen Verriegelungseinrichtung kann ein Sperrstab zur Betätigung der Verriegelungseinrichtung vorgesehen sein. Der Sperrstab kann zum Beispiel mit Hilfe eines elektrischen Antriebes bewegt werden, um die Verriegelungseinrichtung zu entriegeln. Der Antrieb muss dann nur kurze Zeit betrieben werden, bis die Entriegelung stattgefunden hat. Als Antrieb kann z.B. ein Elektromagnet eingesetzt werden.

Eine bevorzugte Ausgestaltung der erfindungsgemäßen Schnellschlussklemme umfasst für den Rückholmechanismus einen Spindeltrieb, der zum Beispiel mit einem Elektromotor angetrieben wird, um die Haltevorrichtung zusammen mit der Klemmbacke aus der Klemmstellung heraus zurückzubewegen.

Eine besonders sichere Ausführungsform umfasst einen Mechanismus, der die durch die Federeinrichtung ausgelöste Bewegung der Klemmbacke begrenzt, also zum Beispiel verhindert, dass die Klemmbacke vollständig aus einem Gehäuse austreten kann. Dazu ist z.B. eine entsprechende Anschlagvorrichtung vorgesehen. Gerade bei transportablen Systemen erhöht dies die Sicherheit signifikant. Unbeabsichtigtes Auslösen der Schnellschlussklemme z.B. während des Transportes kann nicht dazu führen, dass die Klemmbacke durch die Wirkung der Federeinrichtung aus dem Gehäuse vollständig herausgetrieben wird.

Um die Schnellschlussklemme im nicht verwendeten Zustand zu sichern oder um zu verhindern, dass die Schnellschlussklemme ausgelöst wird, obwohl ein Abklemmen gar nicht gewünscht ist, kann eine Feststellvorrichtung zur Festlegung der Klemmbacke und der Haltevorrichtung vorgesehen sein. Die Haltevorrichtung und die Klemmbacke können dazu mit Hilfe der Feststellvorrichtung zum Beispiel in einem Gehäuse festgelegt werden.

Die erfindungsgemäße Schnellschlussklemme kann überall dort zum Einsatz kommen, wo das Abklemmen eines Schlauches notwendig ist. Besonders eignet sich die erfindungsgemäße Schnellschlussklemme für medizinische Geräte, da dort in vielen Fällen ein schneller, sicherer und präziser Abschluss einer Schlauchverbindung notwendig ist.

Die Schnellschlussklemme der Erfindung ist kompakt und einfach im Aufbau. Sie eignet sich daher insbesondere für transportable Systeme.

Die Erfindung wird anhand einer Ausführungsform im Detail erläutert, die in den anliegenden Figuren dargestellt ist. Dabei zeigt:
- Fig. 1: einen seitlichen Schnitt durch eine erfindungsgemäße Schnellschlussklemme,
- Fig. 2: eine seitliche Draufsicht auf eine erfindungsgemäße Schnellschlussklemme,
- Fig. 3: eine Draufsicht in Richtung III, die in Fig. 2 angedeutet ist,
- Fig. 4: ein Detail der Schnittansicht der Fig. 1,
- Fig. 4a: in schematischer Darstellung eine Querschnittsansicht in axialer, in Fig. 4 mit IVa bezeichneter Blickrichtung auf die Sperrstange einer erfindungsgemäßen Ausführungsform, und
- Fig. 5: in schematischer Darstellung ein Detail der erfindungsgemäßen Schnellschlussklemme, das in den Fig. 1 bis 3 nicht dargestellt ist.

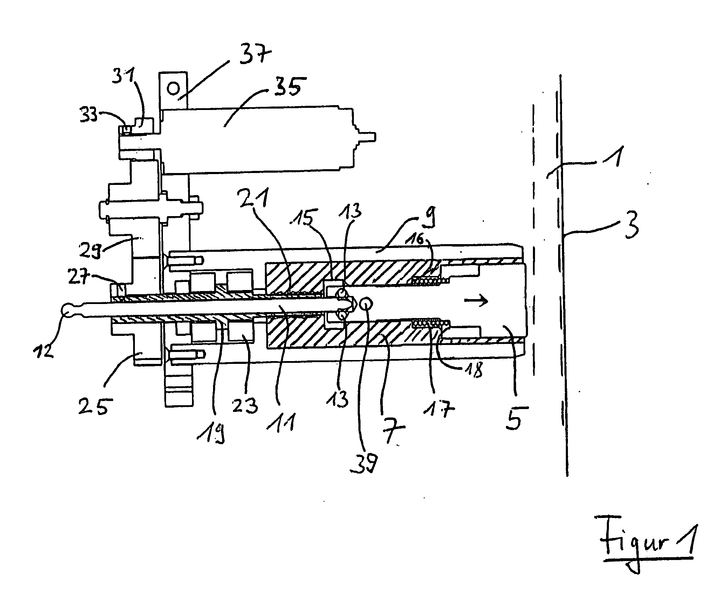

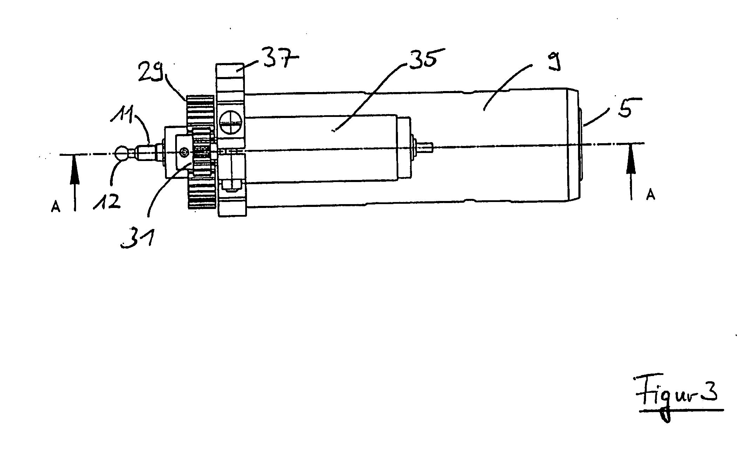

Die Schnittansicht der Fig. 1 entspricht dem in Fig. 3 mit A angedeuteten Querschnitt. 1 bezeichnet einen Schlauch, der ggf. abgeklemmt werden soll, z.B. innerhalb einer Herz-Lungen-Maschine. Sobald in diesem Schlauch z.B. eine Luftblase in dem darin fließenden Blut durch einen nicht gezeigten Detektor erkannt wird, muss die Schlauchklemme den Schlauch abklemmen. Der Schlauch liegt an einer Wand 3 an, die z.B. durch die Rückwand eines den Schlauch aufnehmenden Gehäuseteils gebildet ist. Zum Abklemmen des Schlauches 1 dient eine Klemmbacke 5, die von der Schnellschlussklemme in Pfeilrichtung in eine Klemmstellung gebracht werden muss.

Bezugszeichen 7 bezeichnet eine Haltevorrichtung, die einen inneren Hohlraum aufweist, in dem die Klemmbacke 5 geführt ist. Bei der gezeigten Ausführungsform ist die Klemmbacke 5 als Innenkolben und die Haltevorrichtung 7 als Außenkolben ausgebildet, wobei der Innenkolben 5 in dem Außenkolben 7 und der Außenkolben 7 in einem Gehäuse 9 verschiebbar aufgenommen sind. Gegen einen Ansatz 16 innerhalb des Außenkolbens 7 und einen Ansatz 18 am Außenumfang des Innenkolbens 5 stützt sich eine Feder 17 ab, die in der in der in Fig. 1 gezeigten Offenstellung des Innenkolbens unter Druckspannung ist. 19 bezeichnet die Spindel eines Spindeltriebes, die in dem Bereich, in dem sie in den Außenkolben 7 eingreift, ein Außengewinde 21 aufweist. Der Außenkolben 7 weist ein entsprechendes Gegengewinde am Innenumfang auf, wo er von der Spindel 19 durchsetzt wird. Die Spindel 19 wird in einem Lager 23 drehbar im Gehäuse 9 gehalten. Zum Antrieb der Spindel ist diese mit einem Zahnrad 25 über die Madenschraube 27 verbunden. Das Zahnrad 25 kämmt mit einem Zahnrad 29, das wiederum mit einem Zahnrad 31 kämmt. Das Zahnrad 31 ist über eine Madenschraube 33 mit der Achse eines Elektromotors 35 verbunden, der fest in einer Halteplatte 37 montiert ist. Das Zahnrad 29 ist drehbar in der Halteplatte 37 gelagert. Schließlich ist mit der Halteplatte 37 das Gehäuse 9 fest verschraubt.

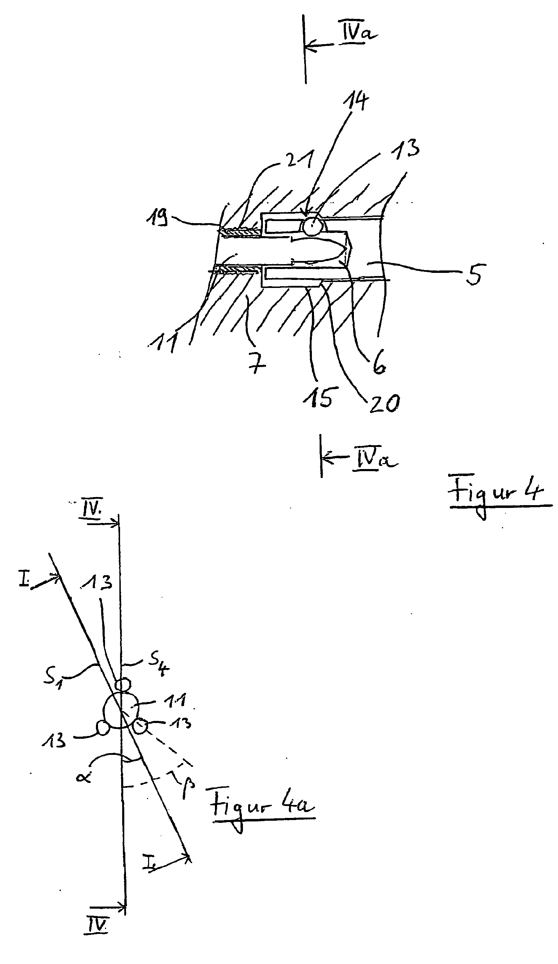

In dem Außenkolben 7 befindet sich ein Hohlraum 15, in den Kugeln 13 teilweise eintreten können, die im verriegelten Zustand aus dem Innenkolben 5 radial herausragen. Dieses Detail wird anhand der Fig. 4 erläutert werden.

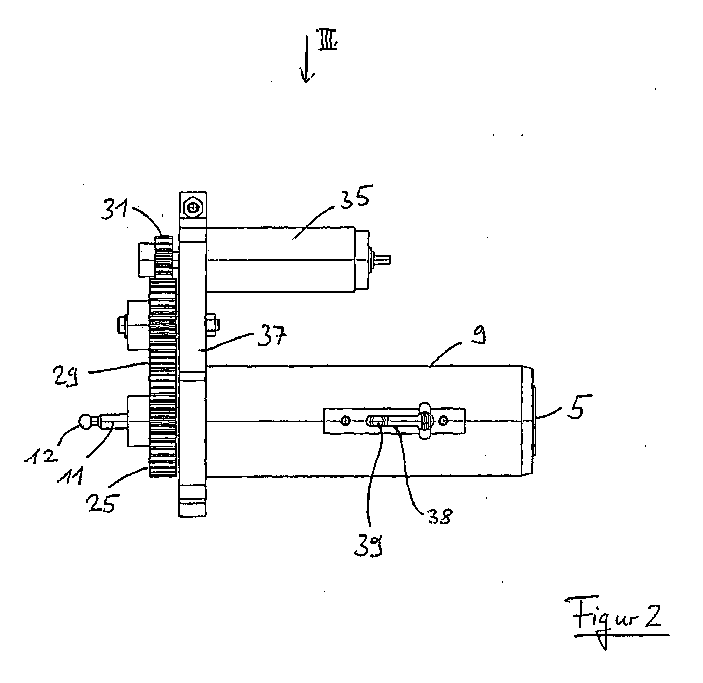

Fig. 2 zeigt die Schnellschlussklemme der Fig. 1 in seitlicher Draufsicht. Mit III ist in Fig. 2 die Blickrichtung angedeutet, die in der Draufsicht der Fig. 3 sichtbar ist. Der Innenkolben 5 umfasst eine sich radial nach außen erstreckende Anschlagstange 39, die in einem Langloch 38 des Gehäuses 9 geführt ist, wie es in Fig. 2 erkennbar ist.

Fig. 4 zeigt ein Detail der Fig. 1 im Bereich der Verriegelungseinrichtung zwischen Außenkolben 7 und Innenkolben 5. Auch in Fig. 4 ist der verriegelte Zustand gezeigt. Der Sperrstab 11 liegt mit seiner Spitze in der axialen Ausnehmung 6 des hinteren Teils des Innenkolbens 5. Die Spitze drückt dabei Kugeln 13 durch radiale Öffnungen 14 im Innenkolben 5 nach außen, die die Kugeln 13 teilweise umfassen. Die Spitze des Sperrstabes 11 ist ballig und nach vorne zugespitzt ausgeführt, so dass sie leicht zwischen die Kugeln 13 geschoben werden kann. Bei der dargestellten Ausführungsform sind von den radialen Öffnungen 14 drei im 120° Winkel zueinander mit entsprechenden Kugeln 13 vorgesehen. In der Schnittdarstellung der Fig. 4 sind die zwei anderen Öffnungen daher nicht sichtbar.

Die Kugeln 13 greifen in eine Ausnehmung 15 in dem Außenkolben 7 ein. Im gezeigten Zustand kann sich dementsprechend der Innenkolben 5 nicht nach rechts aus dem Außenkolben 7 herausbewegen, da die Kugeln 13 in der Ausnehmung 15 des Außenkolbens 7 festgelegt sind. Wird der Sperrstab 11 nach links aus der axialen Ausnehmung 6 des Innenkolbens 5 herausgezogen, so können die Kugeln sich in die axiale Ausnehmung 6 bewegen und der Innenkolben 5 kann durch die Kraft der Feder 17 nach rechts aus dem Außenkolben 7 herausbewegt werden. Die Bewegung der Kugeln 13 nach innen wird insbesondere durch die Anschrägung 20 der Ausnehmung 15 erleichtert.

Schließlich ist in Fig. 4 noch das rechte Ende der Spindel 19 mit dem Gewinde 21 erkennbar, das in einem Innengewinde des Außenkolbens 7 kämmt.

Während Fig. 4 einen Schnitt durch die Schnellschlussklemme zeigt, in dem eine Kugel 13 genau mittig geschnitten wird, zeigt Fig. 1 einen Schnitt, in dem keine Kugel 13 genau geschnitten wird. Insofern sind die Schnittebenen der Fig. 1 und der Fig. 4 um 30° um eine Achse gegeneinander verkippt, die zum Beispiel durch die Sperrstange 11 definiert wird. Dieser Zusammenhang wird in Fig. 4a verdeutlicht, die einen Blick in Richtung der Pfeile IVa zeigt, die in Fig. 4 angegeben sind. In schematischer Darstellung ist in Fig. 4a ein Blick in axialer Richtung auf die Spitze des Sperrstabes 11 und die Kugeln 13 gezeigt. Si zeigt die Schnittebene der Fig. 1, während S₄ die Schnittebene der Fig. 4 zeigt. Die Blickrichtung auf die Schnittebene, die Gegenstand der Fig. 1 ist, ist in Fig. 4a mit den Pfeilen I bezeichnet. Die Blickrichtung auf die Schnittebene, die Gegenstand der Fig. 4 ist, ist in Fig. 4a mit den Pfeilen IV bezeichnet. Der angedeutete Winkel β beträgt 60°, während der Verkippungswinkel der Schnittebenen α 30° beträgt.

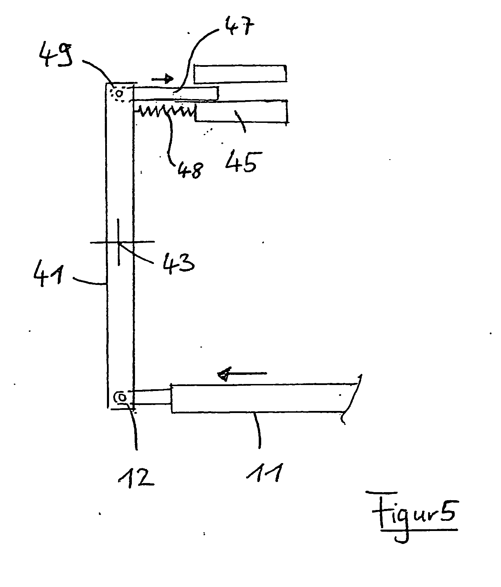

Fig. 5 zeigt in schematischer Darstellung einen Teil der Schnellschlussklemme, der in den Fig. 1 bis 4 nicht dargestellt ist. Es handelt sich dabei um einen Mechanismus, der zur Bewegung der Sperrstange 11 in axialer Richtung dient. Der Sperrstab 11 ist über einen Anlenkpunkt 12 mit einer Wippe 41 verbunden, die am Punkt 43 drehbar gelagert ist. Über einen Anlenkpunkt 49 ist diese Wippe mit einer metallischen Betätigungsstange 47 verbunden, die in einen Elektromagneten 45 ragt. Die Anordnung ist dabei so gewählt, dass sich bei Stromfluss durch den Elektromagneten 45 die Stange 47 nach rechts bewegt. Die Wippe dreht sich um den Drehpunkt 43 und bewegt die Sperrstange 11 in der Darstellung der Fig. 5 nach links.

Bezugszeichen 48 bezeichnet eine Druckfeder, die die Wippe 41, die Betätigungsstange 47 und die Sperrstange 11 in Richtung ihrer Ruhepositionen vorspannt, wenn der Elektromagnet 45 wieder stromlos ist. Die Kraft des Elektromagneten 45 wirkt gegen die Federkraft dieser Feder 48.

Eine erfindungsgemäße Schnellschlussklemme wird wie folgt eingesetzt. Wird im Schlauch 1 durch den nicht gezeigten Detektor eine Blase in der geförderten Flüssigkeit festgestellt, so wird ein Signal an den Elektromagneten 45 gegeben. Der Elektromagnet 45 wird für etwa 50 ms bestromt, so dass sich die Betätigungsstange 47 in den Elektromagneten bewegt. Die Wippe 41 dreht sich um den Drehpunkt 43 und zieht die Sperrstange 11 nach links gemäß der Darstellung der Figuren. Die Sperrstange 11 bewegt sich dadurch aus dem axialen Hohlraum 6 (Fig. 4) des Innenkolbens 5 heraus. Durch die Federkraft der Feder 17 strebt der Innenkolben 5 in Richtung des Pfeils, der in Fig. 1 angegeben ist. Da die Sperrstange 11 den axialen Hohlraum 6 nicht mehr versperrt, weichen die Kugeln 13 dabei - begünstigt durch die Anschrägung 20 - in diesen Hohlraum zurück und die Verriegelung zwischen dem Innenkolben 5 und dem Außenkolben 7 wird aufgehoben. Die Federkraft der Feder 17 treibt den Innenkolben 5 gegen den Schlauch 1 und klemmt ihn gegen die Rückwand 3 des schlauchführenden Kanals ab. Zur Auslösung dieser Aktion reicht es zum Beispiel aus, den Elektromagneten nur über etwa 50 Millisekunden zu betreiben. Bereits nach etwa 100 Millisekunden ist der Schlauch abgeklemmt.

Durch den in dem Langloch 38 in dem Gehäuse 9 geführten Anschlagstab 39 wird verhindert, dass bei unbeabsichtigtem Auslösen der Innenkolben 5 das Gehäuse 9 vollständig verlassen kann.

Um den Innenkolben wieder in seine Offenstellung zu überführen, wird der Elektromotor 35 angeschaltet. Über die Zahnräder 31, 29 und 25 wird die Spindel 19 angetrieben. Der Außenkolben 7 bewegt sich durch die Spindeldrehung axial nach rechts aus dem Gehäuse 9 heraus. Der Innenkolben 5 stützt sich währenddessen weiterhin gegen den Schlauch 1 bzw. die Kanalrückwand 3 ab. Sobald der Außenkolben 7 und der Innenkolben 5 wieder vollständig aufeinandergeschoben sind, sind die radialen Öffnungen 14 in dem Innenkolben 5 wieder im Bereich der Ausnehmung 15 innerhalb des Außenkolbens 7. Die Spitze der Sperrstange 11 kann sich wieder zwischen die Kugeln 13 schieben, die dadurch wiederum durch die Öffnungen 14 im Innenkolben 5 radial nach außen bewegt werden. Die Sperrstange 11 wird dazu durch die Wirkung der Feder 48, die auf die Wippe 41 wirkt, in die axiale Ausnehmung 6 des Innenkolbens 5 eingeschoben. Die Kugeln 13 greifen wieder in die Ausnehmung 15 im Außenkolben 7 ein, wie es in Fig. 4 gezeigt ist und verriegeln den Außenkolben 7 und den Innenkolben 5.

Wird der Elektromotor 35 jetzt in umgekehrter Richtung betrieben, zieht die Spindel 19 den Außenkolben 7 in der Darstellung der Figuren nach links. Auf Grund der Verriegelung des Innenkolbens 5 in dem Außenkolben 7 wird auch der Innenkolben 5 zurück bewegt und die Schnellschlussklemme kommt wieder in ihre Offenstellung.

Bereits während der Außenkolben 7 wieder über den Innenkolben 5 geschoben wird, spannt sich die Feder 17 erneut an und speichert Energie für einen neuen Auslösevorgang. Auf diese Weise ist es möglich, dass bereits beim Zurückholen des Innenkolbens 5 zusammen mit dem Außenkolben 7 bei Bedarf ein weiterer Abklemmvorgang ausgelöst wird, wenn z.B. bereits während der Rückführung des Innenkolbens 5 erneut eine Blase in dem extrakorporalen Kreislauf detektiert wird.

Die erfindungsgemäße Schnellschlussklemme eignet sich auf Grund ihres kompakten und integralen Aufbaus insbesondere für transportable Systeme.

### Bezugszeichenliste

- 1: Schlauch
- 3: Kanalwand
- 5: Klemmbacke, Innenkolben
- 6: axiale Ausnehmung am rückwärtigen Ende des Innenkolbens
- 7: Haltevorrichtung, Außenkolben
- 9: Gehäuse
- 11: Sperrstab
- 12: Anlenkpunkt
- 13: Kugeln
- 14: radiale Öffnungen
- 15: Ausnehmung in dem Außenkolben
- 16: Ansatz in dem Außenkolben
- 17: Feder
- 18: Ansatz an dem Innenkolben
- 19: Spindel
- 20: Anschrägung in der Ausnehmung des Außenkolbens
- 21: Gewinde
- 23: Lager
- 25, 29, 31: Zahnräder
- 27,33: Madenschrauben
- 35: Elektromotor
- 37: Halteplatte
- 38: Langloch
- 39: Anschlagstange
- 41: Wippe
- 43: Wippendrehpunkt
- 45: Elektromagnet
- 47: Betätigungsstange
- 48: Feder
- 49: Anlenkpunkt

## Patentansprüche

1. Schnellschlussklemme zum Abklemmen von Schläuchen, die folgendes aufweist:
a) eine Klemmbacke (5) mit einer Klemmstellung, in der sie einen Schlauch (1) abklemmen kann, und einer Offenstellung,
b) eine bewegliche Haltevorrichtung (7),
c) eine Federeinrichtung (17), die die Klemmbacke (5) gegen die Haltevorrichtung (7) derart vorspannt, dass die Klemmbacke (5) bei Entspannung der-Federeinrichtung (17) in die Klemmstellung gebracht wird,
d) eine Verriegelungseinrichtung (11, 13), mit der die Klemmbacke (5) und die Haltevorrichtung (7) gegen die Federkraft der Federeinrichtung (17) in einer Verriegelungsstellung lösbar miteinander verriegelt werden können, und
e) einen Rückholmechanismus (19) zur Bewegung der Haltevorrichtung (7) relativ zu der Klemmbacke (5) bis die Haltevorrichtung (7) und die Klemmbacke (5) in ihrer Verriegelungsstellung zueinander sind, während die Klemmbacke (5) in der Klemmstellung ist,
und zur Bewegung der Haltevorrichtung (7) zusammen mit der Klemmbacke (5) in die Offenstellung der Klemmbacke (5), nachdem die Klemmbacke und die Haltevorrichtung (7) mit der Verriegelungseinrichtung miteinander verriegelt worden sind.

2. Schnellschlussklemme nach Anspruch 1,
bei der die Haltevorrichtung ein Hohlteil (7) umfasst, in dem die Klemmbacke (5) geführt ist.

3. Schnellschlussklemme nach Anspruch 2,
bei der die Federeinrichtung (17) eine sich einerseits gegen einen Ansatz (16) innerhalb des Hohlteils (7) und andererseits gegen einen Ansatz (18) in der Klemmbacke (5) abstützende Feder (17) umfasst.

4. Schnellschlussklemme nach einem der Ansprüche 1 bis 3,
bei der die Verriegelungseinrichtung (11, 13) mechanisch ist.

5. Schnellschlussklemme nach Anspruch 4,
bei der die Verriegelungseinrichtung zumindest ein Element (13) umfasst, das im Verriegelungszustand seitlich aus der Klemmbacke (5) in eine Ausnehmung (15) der Haltevorrichtung (7) eingreift, und in einem Entriegelungszustand die Ausnehmung (15) freigibt.

6. Schnellschlussklemme nach Anspruch 5,
bei der das zumindest eine Element eine Kugel (13) ist, die im Verriegelungszustand durch eine radiale Öffnung (14) in die Ausnehmung (15) der Haltevorrichtung (7) vorsteht, wobei die Öffnung (14) die Kugel (13) teilweise umfasst, und im Entriegelungszustand die Kugel nicht aus der Öffnung (14) vorsteht.

7. Schnellschlussklemme nach einem der Ansprüche 1 bis 6,
bei der die Verriegelungseinrichtung einen Sperrstab (11), der die Verriegelungseinrichtung betätigt, und einen elektrischen Antrieb zur Bewegung des Sperrstabes (11), vorzugsweise einen Elektromagneten (45) umfasst.

8. Schnellschlussklemme nach Anspruch 7,
bei der der elektrische Antrieb (45) derart ausgestaltet und angeordnet ist, dass ein Bestromen des Antriebes (45) zu einer Bewegung des Sperrstabes (11) führt, die zu einer Entriegelung der Verriegelungseinrichtung führt.

9. Schnellschlussklemme nach einem der Ansprüche 1 bis 8,
bei der der Rückholmechanismus einen Spindeltrieb (19) umfasst.

10. Schnellschlussklemme nach einem der Ansprüche 1 bis 9,
mit einer Anschlagvorrichtung (38, 39), die derart ausgestaltet ist, dass sie die durch die Federeinrichtung (17) ausgelöste Bewegung der Klemmbacke (5) begrenzt.

## Claims

1. A fast closing clamp for the pinching off of hoses comprising the following:
a) a clamp jaw (5) with a clamping position, in which it can pinch off a hose (1), and an open position;
b) a movable holding apparatus (7);
c) a spring device (17) which biases the clamp jaw (5) with respect to the holding apparatus (7) such that the clamp jaw (5) is brought into the clamping position on the relaxation of the spring device (17);
d) a latch device (11, 13) with which the clamp jaw (5) and the holding apparatus (17) can be releasably latched to one another against the spring force of the spring device (17) in a latched position; and
e) a return mechanism (19) for the movement of the holding apparatus (7) relative to the clamping jaw (5) until the holding apparatus (7) and the clamp jaw (5) are in their latched position relative to one another while the clamp jaw (5) is in the clamping position,
and for the movement of the holding apparatus (7) together with the clamp jaw (5) into the open position of the clamp jaw (5) after the clamp jaw and the holding apparatus (7) have been latched to one another using the latching device.

2. A fast closing clamp in accordance with claim 1, wherein the holding apparatus comprises a hollow part (7) in which the clamp jaw (5) is guided.

3. A fast closing clamp in accordance with claim 2, wherein the spring device (17) comprises a spring (17) supported, at the one end, against a seat (16) inside the hollow part (7) and, at the other end, against a seat (18) in the clamp jaw (5).

4. A fast closing clamp in accordance with any one of the claims 1 to 3, wherein the latch device (11, 13) is mechanical.

5. A fast closing clamp in accordance with claim 4, wherein the latch device comprises at least one element (13) which engages laterally from the clamp jaw (5) into a cut-out (15) of the holding apparatus (7) in the latched state and frees the cut-out (15) in an unlatched state.

6. A fast closing clamp in accordance with claim 5, wherein the at least one element is a ball (13) which projects through a radial opening (14) into the cut-out 15) of the holding apparatus (7) in the latched state, with the opening (14) partly encompassing the ball (13) and the ball not projecting out of the opening (14) in the unlatched state.

7. A fast closing clamp in accordance with any one of the claims 1 to 6, wherein the latch device comprises a blocking bar (11) which actuates the latch device and an electrical drive for the movement of the blocking bar, (11) preferably an electromagnet (45).

8. A fast closing clamp in accordance with claim 7, wherein the electrical drive (45) is designed and arranged such that a current flow to the drive (45) results in a movement of the blocking bar (11) which results in an unlatching of the latch device.

9. A fast closing clamp in accordance with any one of the claims 1 to 8, wherein the return mechanism comprises a spindle drive (19).

10. A fast closing clamp in accordance with any one of the claims 1 to 9, having an abutment device (38, 39) which is designed such that it bounds the movement of the clamp jaw (5) triggered by the spring device (17).

## Revendications

1. Pince à fermeture rapide pour obturer des tuyaux souples, comprenant les éléments suivants :
a) une mâchoire de serrage (5) avec une position de serrage, dans laquelle elle peut obturer un tuyau (1), et une position d'ouverture,
b) un dispositif de maintien déplaçable (7),
c) un dispositif à ressort (17), qui précontraint la mâchoire de serrage (5) contre le dispositif de maintien (7) de telle façon que la mâchoire de serrage (5) est amenée dans la position de serrage lors de la détente du dispositif à ressort (17),
d) un dispositif de verrouillage (11, 13) au moyen duquel la mâchoire de serrage (5) et le dispositif de maintien (7) peuvent être verrouillés l'un à l'autre à l'encontre de la force élastique du dispositif à ressort (17) dans une position de verrouillage et de façon libérable, et
e) un mécanisme de rappel (19) pour déplacer le dispositif de maintien (7) par rapport à la mâchoire de serrage (5) jusqu'à ce que le dispositif de maintien (7) et la mâchoire de serrage (5) soient l'un vers l'autre dans leur position de verrouillage, alors que la mâchoire de serrage (5) est dans la position de serrage,
et pour déplacer le dispositif de maintien (7) conjointement avec la mâchoire de serrage (5) jusque dans la position d'ouverture de la mâchoire de serrage (5), après que la mâchoire de serrage et le dispositif de maintien (7) aient été verrouillés l'un à l'autre avec le dispositif de verrouillage.

2. Pince à fermeture rapide selon la revendication 1,
dans laquelle le dispositif de maintien comprend une pièce creuse (7)
dans laquelle la mâchoire de serrage (5) est guidée.

3. Pince à fermeture rapide selon la revendication 2,
dans laquelle le dispositif à ressort (17) comprend un ressort (17) qui s'appuie d'une part contre un talon (16) à l'intérieur de la pièce creuse (7) et d'autre part contre un talon (18) dans la mâchoire de serrage (5).

4. Pince à fermeture rapide selon l'une des revendications 1 à 3,
dans laquelle le dispositif de verrouillage (11, 13) est mécanique.

5. Pince à fermeture rapide selon la revendication 4,
dans laquelle le dispositif de verrouillage comprend au moins un élément (13) qui, dans l'état verrouillé, s'engage latéralement hors de la mâchoire de serrage (5) jusque dans un évidement (15) du dispositif de maintien (7) et qui, dans l'état déverrouillé, libère l'évidement (15).

6. Pince à fermeture rapide selon la revendication 5,
dans laquelle ledit au moins un élément est une bille (13) qui, dans l'état verrouillé, dépasse à travers une ouverture radiale (14) jusque dans l'évidement (15) du dispositif de maintien (7), ladite ouverture (14) entourant partiellement la bille (13), et dans l'état déverrouillé, la bille ne dépasse pas hors de l'ouverture (14).

7. Pince à fermeture rapide selon l'une des revendications 1 à 6,
dans laquelle le dispositif de verrouillage comprend une barre de blocage (11) qui actionne le dispositif de verrouillage, et un entraînement électrique pour déplacer la barre de blocage (11), de préférence à un électroaimant (45).

8. Pince à fermeture rapide selon la revendication 7,
dans laquelle l'entraînement électrique (45) est conçu et agencé de telle façon qu'une alimentation électrique de l'entraînement (45) mène à un déplacement de la barre de blocage (11), qui mène un déverrouillage du dispositif de verrouillage.

9. Pince à fermeture rapide selon l'une des revendications 1 à 8, dans laquelle le mécanisme de rappel comprend un entraînement à broche (19).

10. Pince à fermeture rapide selon l'une des revendications 1 à 9,
comprenant un dispositif à butée (38, 39) qui est conçu de telle façon qu'il limite le mouvement de la mâchoire de serrage (5) déclenché par le dispositif à ressort (17).
